# EUROPEAN PATENT SPECIFICATION

(11) **EP 3 764 872 B1**
(45) Date of publication and mention of the grant of the patent: **19.01.2022**
(21) Application number: 19711586.8
(22) Date of filing: 15.03.2019
(51) Int. Cl.: A61B 5/00, A61F 2/844, A61B 5/026, A61B 5/02, A61F 2/82

(54) **DEVICE FOR INTRAVASCULAR MONITORING**
VORRICHTUNG ZUR INTRAVASKULÄREN ÜBERWACHUNG
DISPOSITIF DE SURVEILLANCE INTRAVASCULAIRE

(30) Priority: 16.03.2018 EP 18162366
(43) Date of publication of application: 20.01.2021
(73) Proprietor: Koninklijke Philips N.V., 5656 AG Eindhoven (NL)
(72) Inventor: HENDRIKS, Cornelis, Petrus, 5656 AE Eindhoven (NL); HILGERS, Achim, Rudolf, 5656 AE Eindhoven (NL); MUELLER, Manfred, 5656 AE Eindhoven (NL); VAN DER HORST, Arjen, 5656 AE Eindhoven (NL); JOHNSON, Mark, Thomas, 5656 AE Eindhoven (NL); HAKKENS, Franciscus, Johannes, Gerardus, 5656 AE Eindhoven (NL); VAN DEN ENDE, Daan, Anton, 5656 AE Eindhoven (NL)
(74) Representative: Philips Intellectual Property & Standards
(86) International application number: PCT/EP2019/056574
(87) International publication number: WO 2019/175401

(56) References cited:
- WO-A2-2010/019773
- US-A1- 2015 320 357
- US-A1- 2016 022 447

## Description

### FIELD OF THE INVENTION

This invention relates to devices for in-body monitoring. In particular, for monitoring blood flow and/or vessel obstruction.

### BACKGROUND OF THE INVENTION

There is increasing demand for unobtrusive health sensing systems. In particular, there is a shift from conventional hospital treatment towards unobtrusive vital signs sensor technologies, centered around the individual, to provide better information about the subject's general health.

Such vital signs monitor systems help to reduce treatment costs by disease prevention and enhance the quality of life. They may provide physiological data for physicians to analyze when attempting to diagnose a subject's general health condition. Vital signs monitoring typically includes monitoring one or more of the following physical parameters: heart rate, blood pressure, respiratory rate and core body temperature.

By way of example, in the US about 30% of the adult population has a high blood-pressure. Only about 52% of this population have their condition under control. Hypertension is a common health problem which has no obvious symptoms and may ultimately cause death, and is therefore often referred to as the "silent killer". Blood pressure generally rises with aging and the risk of becoming hypertensive in later life is considerable. About 66% of the people in age group 65-74 have a high blood pressure. Persistent hypertension is one of the key risk factors for strokes, heart failure and increased mortality.

The condition of the hypertensive patients can be improved by lifestyle changes, healthy dietary choices and medication. Particularly for high risk patients, continuous 24-hour blood pressure monitoring is very important and there is obviously a desire for systems which do not impede ordinary daily life activities.

It is known to provide implantable sensor devices for monitoring physiological parameters, such as blood pressure. While the initial insertion is an invasive procedure, once this is completed, the sensor remains in place for unobtrusive sensing for a prolonged period of time. Thus, implanted sensor technologies are also seen as minimally invasive (in the long term).

Implantable devices enable unobtrusive and long term monitoring of patients with chronic diseases such as heart failure, peripheral artery disease or hypertension. The purpose of monitoring is to provide reassurance or early warning indicators, but also to reduce, or in general, to control medication.

This invention relates more particularly to the monitoring of intravascular conditions after insertion of a stent or other vascular implant. In general, there is a need to monitor the formation of a bio-layer in a vessel after stent placement to see if new problems might occur and to help in decisions on new interventions or medication use.

The nature of the bio-layer can be different, for example restenosis, thrombus, hyperplasia, endothelial overgrowth or plaque. However, the monitoring of bio-layer formation alone is not sufficient to decide if there is a problem or not. Instead, the blood flow conditions ideally should also be monitored. Measuring flow may additionally provide information on upstream hemodynamic conditions (for example to determine that a stent is open but has no flow because of an upstream occlusion).

Stent patency is the state of being open and unblocked. For example, a common problem with coronary stents is "in-stent restenosis" (ISR), even with drug eluting stents. Follow-up after treatment is commonly implemented with computer tomography imaging. The disadvantages are the need for an X-ray dose, the need for a hospital visit, and the fact that it is only a point measurement in time. Instead it would be desirable to continuously monitor stent patency and to provide an early warning when necessary, or to reduce medication when possible.

There are known implantable blood pressure sensors (e.g. by from the company Cardiomems ^{™}), and restenosis sensors (from the company Instent ^{™}- based on measurement of an impedance change as a function of restenosis), as well as actuators for controlled drug delivery such as a micro-peristaltic pump (from the company MPS microsystems).

By way of further example, stents with blood flow sensors are described in US 2005/0277839A1 based on a surface acoustic wave flow sensor, and WO 1998/029030A1 based on electrode impedance/conductance measurements.

WO 2010/019773 discloses smart stent systems and methods for evaluating fluid flow through a stent. The systems comprise a stent having a coil configured as a resonant circuit and a reader. During use, an oscillation is produced in the coil of the stent by applying an RF field to the stent. Then, a parameter associated with the oscillation is obtained to evaluate fluid flow through the stent.

US 2015/320357 discloses a method for assessing fluid flow in a conduit. The method includes sensing the fluid flow with a sensor and generating data from the sensor that relates to the sensed fluid flow. The data is output from the sensor and filtered so that it may be interpreted to characterize the fluid flow. The conduit may be a prosthesis, such as a stent, and the flow may be any bodily fluid, such as blood, bile, or cerebrospinal fluid.

US 2010/087782 discloses a system, device, and method for utilizing stretchable active integrated circuits with inflatable bodies. This allows operative features to come into direct contact with body structures, such as the inner wall of a lumen. Such direct contact increases accuracy of measurement and delivery of therapy.

US 2016/022447 discloses a stent including a wire tube and at least one pressure sensor in electrical contact with the wire tube. The pressure sensor includes a diaphragm in communication with a reservoir of liquid, a channel in fluid communication with the reservoir, and at least one pair of electrodes disposed on opposite sides of the channel.

### SUMMARY OF THE INVENTION

It is a problem of many sensor designs that they no longer function if they are overgrown with a bio-layer (even at a very limited bio-layer depth, e.g. 100 micrometers).

Such sensors are also typically positioned near the lumen wall which impairs the sensing ability.

There is no current sensor design available which is able to measure reliably both flow and bio-layer formation over time. In particular, known flow sensors are not able to function after bio-layer formation. It would accordingly be desirable to have a single sensor which is simple and reliable and which can measure blood flow even if there is some bio-layer formation. A further goal would be to have a sensor that can also measure the bio-layer formation.

The invention is defined by the independent claim.

According to examples in accordance with an aspect of the invention, there is provided a monitoring system comprising:
- an intra-vascular support device having a length direction, for positioning against a vessel wall in use, with its length direction aligned with a vessel direction; and
- a sensor device mounted to the intra-vascular support device,
wherein the sensor device comprises or consists of:
- a deformable part that is spaced from the intra-vascular support device such that when a flowing medium flows along the support device, the deformable part at least partly extends perpendicularly to the length direction of the intra-vascular support device into the flowing medium, the sensor being adapted to generate a sensor signal which is dependent on a deformation or deformability of the deformable part.

The intra-vascular support device and the sensor device may be part of a catheter or an implantable unit.

The system is for placement in a vessel or artery or other lumen wherein a flowing medium resides. The deformable part is thus spaced from the support device such that it at least partly extends into the medium when the system is in use. A flow of the medium may thus cause deformation or movement (vibration) of the sensor and by sensing one or more of these properties a level of flow may be detected or determined.

Furthermore, since the sensor is spaced from the support device a possible growth of a bio-layer on the support device and/or the sensor may be detected too. After all, this bio-layer will influence the deformation or movement (vibration) of the sensor too and such may be determined with the device at hand.

Thus, the physical property preferably comprises the thickness of the bio-layer, preferably at the location of the sensor device. Alternatively or additionally, the property comprises a mechanical property such as rigidity, stiffness of the bio-layer and/or a compositional property.

This system thus makes use of a sensor of which at least a part is flexible/deformable and which with at least part of its deformable part projects into a vessel when the sensor is in the vessel. Thus, the deformable part extends at least partly into a flowing medium (e.g. blood) that forms part of the content of the vessel. Since the sensor signal is dependent on the deformability of the sensor, and due to its arrangement the deformability may be influenced by bio-film formation around and/or on the senor, it is possible to detect whether bio-film formation is occurring and possibly also to what extent. The capability of the sensor to detect the presence of the bio-layer is based on the fact that part of the sensor becomes more rigid or less deformable when constrained by the bio-layer formed in its vicinity. This alters the sensor characteristics and this can be detected.

The system comprises a controller adapted to:
- receive the sensor signal; and
- determine from the received sensor signal:
   - an indication for a level of flow of the flowing medium; and/or
   - an indication of a level of possible bio-layer formation on the intravascular support device and/or the sensor device and/or an indication of a physical property of the bio-layer.

The controller can be adapted to determine the indication of level of flow of the flowing medium based on the deformation or deformability (stiffness) of the deformable part of the sensor.

There may also be a monitoring device as part of the system, which device comprises a user interface for providing any one of the indications to a user.

The system of the invention is thus capable of determining the extent to which an implantable device (or any other device that remains in vessels such as catheters or drains) is still open, i.e. allows flow through of the liquid normally flowing through the vessel, as any bio-film formation within or on such device may reduce such openness. This is an important indication for implantable devices such as stents of many kinds.

The arrangement of the sensor offers a further effect in that because of its arrangement, it may be used to measure the flow of liquid through along the sensor and within the vessel even when a part of the sensor is covered or embedded within a bio-film layer. This extra function may be implemented using the same sensor and having a controller that is capable of determining from the sensor signal a flow of medium along the sensor (i.e. the flow of liquid through the vessel. Thus, the sensor is not only able to provide indication on bio-film formation, but also to detect a flow of medium in the vessel even when bio-film layer formation occurs to some extent.

The sensor and support device can be part of a catheter type device. Alternatively, the sensor and support, and optionally the control device, can be implantable devices. Such devices can have a tubular shape such as a cylindrical shape. The support can be for positioning against the wall of the vessel.

Thus, a single implantation procedure is needed to enable monitoring of both physiological conditions/parameters, i.e. flow and bio-layer formation in a vessel.

By bio-layer formation may in general be meant formation of a semi-solid or solid layer of biological material e.g. on a part of the surface of the sensor and/or support device. Examples of such layers include for instance restenosis, thrombus, hyperplasia, endothelial overgrowth or plaque.

A level of bio-layer formation may in examples refer to a thickness of a bio-layer which forms, or an extent of bio-layer formation along a length of the free part of the sensor for instance.

By 'length of the deformable part' is meant the length of the sensor which is not constrained by the bio-layer, i.e. a free length, or an effective free length, because the bio-layer may not render the constrained portion of the beam to be fully rigid. This system is thus adapted to determine the length of a deformable part of the sensor at a particular time. This length will be influenced and dependent on the level of a bio-layer present and hence from the original length of the sensor and the determined length an estimate of a level or thickness of the bio-layer may be obtained.

The controller may be part of the implantable device, or it may be an external part, or else the controller functions may be divided between implanted components and non-implanted components.

The support device for example comprises a stent. A stent is susceptible to formation of a bio-layer, and this can be monitored by the sensor, which is an integral part of the stent.

The length of the sensor is for example between 0.4 and 0.7 times a diameter of the stent or graft. Thus, it projects at least partly into the cross sectional area of the vessel in which it is implanted.

The sensor for example comprises a capacitance sensor. Bending of the sensor then induces a capacitance change which can be measured. The sensor may comprise an electroactive polymer sensor. The electroactive polymer sensor may comprise a body partially or completely formed of electroactive polymer material, deformable in response to electrical stimulation.

Electroactive polymer material sensor-actuators have the advantage of mechanically simple construction and functionality. This contrasts for instance with mechatronic or other electromechanical actuators or sensors. EAPs also allow small form factor, ideal for deployment in blood vessels, where avoiding occlusion of the vessel is important. They also have long lifetime, limiting the need for future invasive procedures to replace the device.

By way of example, the sensor may comprise an ionic polymer membrane sensor. These are low voltage devices suitable for in-body operation.

According to the invention, the controller is adapted to detect changes in length based on detecting changes in a resonant frequency of the sensor. The natural resonance depends on the free length of the sensor and is thus dependent on the level of bio-layer formation in the vessel.

The controller may for example determine a resonant frequency based on capacitance changes in a frequency range above a frequency range of the capacitance variation caused by the flow being monitored. The resonant frequency is typically higher than the frequency components of interest of the flow detection signal. Thus, by separating the sensor response by frequency, the flow detection and layer detection functions can be separated.

The controller may be adapted to determine a resonant frequency based on resonance induced by the flow being monitored. Thus, the sensor may have a resonance component of its response which is induced by the flow in which it is situated.

Alternatively, the system may further comprise an actuator for actuating the sensor, wherein the controller is adapted to determine a resonant frequency based on resonance induced by the actuation. In this way, the sensor is driven to oscillate at its resonant frequency. The sensing and actuation functions may be performed by the same device, for example when an electroactive polymer sensor-actuator is employed. Alternatively, separate actuation and sensing mechanisms may be employed.

The system may also comprise an actuator for actuating the sensor at an ultrasound frequency for implementing a sensor cleaning function. This helps to maintain a constant sensing characteristic. The sensing and actuation functions may again be performed by the same device.

The controller is for example adapted to determine a level of flow based on the amplitude of the sensor signal, in particular at low frequencies below the resonant frequency.

The system may further comprise a look up table for providing a first mapping between sensor signals and changes in length and a second mapping between sensor signals and a level of flow. This look up table may be populated by a calibration routine. Alternatively, algebraic functions may be used to map between sensor readings and the read out parameters (flow and bio-layer thickness).

The system may comprise a plurality of sensors at different locations on the support device. This enables measurements at different locations. There may be sensors at different circumferential positions and/or different axial positions.

The sensor may have a non-deployed folded state for use during implantation of the system and a deployed unfolded state for subsequent use in monitoring a subject. It is thus implanted as part of a known implantation process for the support device, e.g. stent.

### BRIEF DESCRIPTION OF THE DRAWINGS

Examples of the invention will now be described in detail with reference to the accompanying schematic drawings, in which:
Figs. 1A and 1B show a sensor system located in a vessel; and outside of a vessel.
Fig. 2 shows the control circuit in more detail.
Figs. 3A and 3B show sensor systems with multiple sensors along the support length direction.
Figs. 4A to 4D show sensor systems with multiple sensors at different axial positions.
Figs. 5A to 5C show sensor systems with multiple sensors at different axial positions.
Figs. 6A to 6F show sensor systems with sensors fixed with both ends to a support.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a monitoring system which comprises an intra-vascular support device and a sensor mounted to the support device and projecting from the support device. Thus, when the device is in use and placed in a vessel, the sensor projects into the contents of the vessel. The sensor generates a signal which is dependent on the level of deformation or deformability of a free end of the sensor. The sensor signal is interpreted to enable detection of changes in the length of a deformable part of the sensor thereby to determine a level of bio-layer formation and also determine a level of flow of contents within the vessel. The sensor remains able to detect flow even when a bio-layer is formed and it can also detect the presence (and thickness) of the bio-layer, because part of the sensor becomes rigid when constrained by the bio-layer.

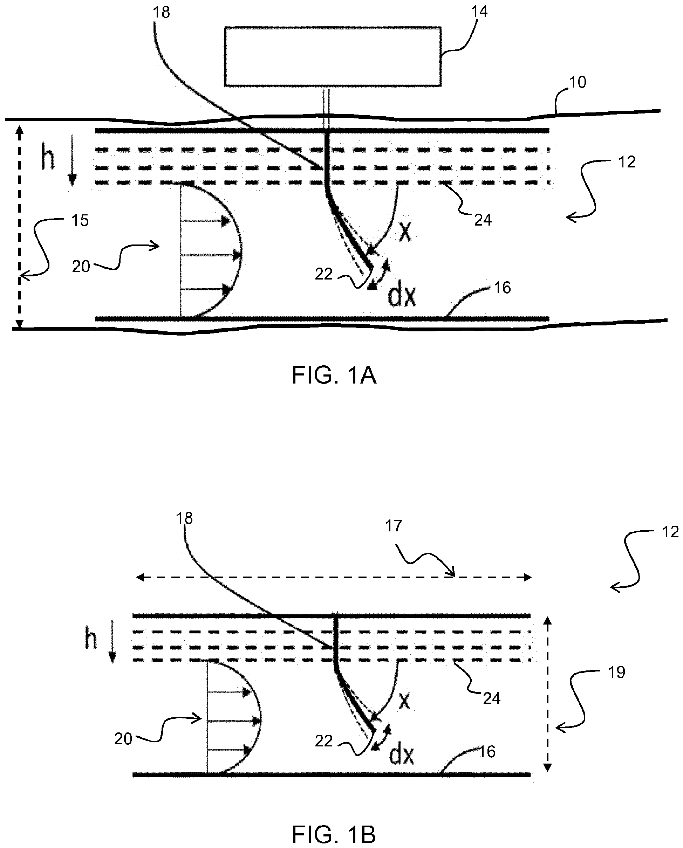

Fig. 1B shows a sensor system and Fig. 1A shows the same sensor system located in a vessel 10. In this case the system is implantable such as with a stent type device, but this need not be the case. It may be a system that is positioned during a treatment period (for example in a hospital) after which it is removed again. The system can thus be part of a catheter type device. The sensor system has a physical sensor part 12 and a controller 14. The sensor part 12 comprises an intra-vascular support device 16 having a length 17 along a length direction. The device is for positioning against the vessel wall with its length direction aligned with the vessel's axial direction as shown. In this case the support device 16 has a tubular shape along the length direction, but other shapes are possible. In this case the support device has a cylindrical shape with a diameter 19 comparable to that of the diameter 15 of the vessel in order to facilitate the positioning.

Within the support device, when the system is in use in the vessel, the support device bounds a space or volume through which a content of the vessel (such as for example blood) can flow. In this space a sensor 18 is located. The sensor 18 is mounted to the support device and projects substantially perpendicularly to the length direction, i.e. across the cross sectional area of the space or volume and therewith it projects into the vessel content when flowing through or along the support device. The vessel is typically an artery and a blood flow is represented as 20 which is along the axial direction of the vessel. The sensor 18 has a length L measured from the point of mounting to the support to its free end 22. The sensor 18 is deformable (e.g. bendable) perpendicular to its length direction. A sensor signal generated by the sensor is generally dependent on the level of deformation of the sensor. As such the sensor 18 is capable of sensing the flow 20 as the flow 20 will lead to a deformation of the shape of the sensor.

The sensor is designed (flexible enough e.g. by choice of dimensions and materials) such that it undergoes a general deformation x as function of the flow speed, but with superposed higher frequency oscillations dx which result from a resonant oscillation of the sensor.

The controller 14 is adapted to receive the sensor signals. This can be via wired connection to the sensor, but can also be via wireless connection such as capacitive or inductive coupling or other signal transfer methods. Wireless connection may be preferred if the controller is to be placed outside the vessel as then no puncture of the vessel needs to occur. Wired connection can be used if the controller is mounted to the support device, or is outside the body and connected via wiring placed within the vessel.

There are various options for the amount of data storage and processing capability which is implanted and the amount that is external. At one extreme, the implanted sensor is simply a passive resonant circuit, which is energized by an external controller. The sensor output is then the impedance of the circuit which can be probed remotely. In this case, there is no storage of sensor data, and a sensor signal is only obtained when an external controller is used to probe the sensor. At the other extreme, the implanted sensor includes a local energy source, such as a battery, and a controller which stores sensor readings over time, processes them to derive the desired output information, and sends them using a wireless transmitter when interrogated by an external transmitter device. In this case, there can be continuous monitoring and storage of sensor data. Any suitable wireless transmission technology may then be used, including RF transmission or other shorter range impedance coupling techniques, described in further detail below.

The analysis of the sensor signal by the controller 14 enables changes in the length of a deformable part of the sensor 18 to be detected thereby to determine a level (e.g. height h) of formation of a bio-layer 24. Additionally, or alternatively, this configuration allows measurement of a level of the flow 20 even if a bio-layer 24 has been formed. Thus the sensor in an example can do either of the two or both.

The sensor is any sensor device which generates a signal which varies in a predictable way as a function of a degree of bending and/or a degree of deformability of the sensor. Such signal can be any signal that can be measured, but a suitable signal is an electrical signal such as voltage, current or impedance. To generate such signals, the sensors in general have a structure that defines an electrical element that has characteristics that are dependent on the deformation and that can be (electrically or otherwise) measured. Thus, for example such devices can have electrodes and a deformable dielectric that are part of an electrical capacitor of which the capacitance is dependent on the deformation.

A preferred example of a sensor is a flexible electroactive device that is used in passive form. In general, these can have an electroactive material sandwiched between electrodes. Such sensors indeed have a variable impedance such as a variable capacitance as described hereinabove. In the example of Figs. 1A and 1B this sensor comprises or consists of a passive electroactive polymer (EAP) sensor. A useful one can be an ionic polymer membrane sensor as it operates at low voltage and is suitable for both sensing and actuation within an in-body environment. The sensor 18 is in one example an ionic polymer membrane (e.g. IPMC) electroactive polymer sensor, with flexible electrodes on both sides. The use of such sensors as flow sensors is known. However, a different type of deformable capacitance sensor may be used. Thus, other such sensors can be used and many of such sensors have been described in prior art and a number of examples is described herein below.

When in an artery or vessel, the sensor may deform and vibrate due to varying hydrodynamic forces in the cardiac cycle therewith causing the varying electrical signals.

The frequency or damping of the vibrations (dX) depends on the free length of the sensor (defined by the difference between the original sensor length L and the bio-layer thickness h) which is the part of the sensor length that is not buried within the bio-layer and which depends on the thickness h of the bio-layer 24. The vibrations cause a rapidly varying impedance and/or capacitance of the free part of the sensor.

Also, the deformation x depends on the blood flow velocity which varies with the cardiac cycle and across the blood vessel. The deformation x causes a slowly varying capacitance.

The timescale differences between the deformations can be used to separate their origins and provide separate information on blood flow and bio-layer formation. The mechanical properties of the sensor are then optimized such that it can bend and vibrate at the same time and in a force range that is suitable for measuring the vibrations and/or the flow 20 in for an application at hand, such as a stent or implantable in a blood vessel or artery.

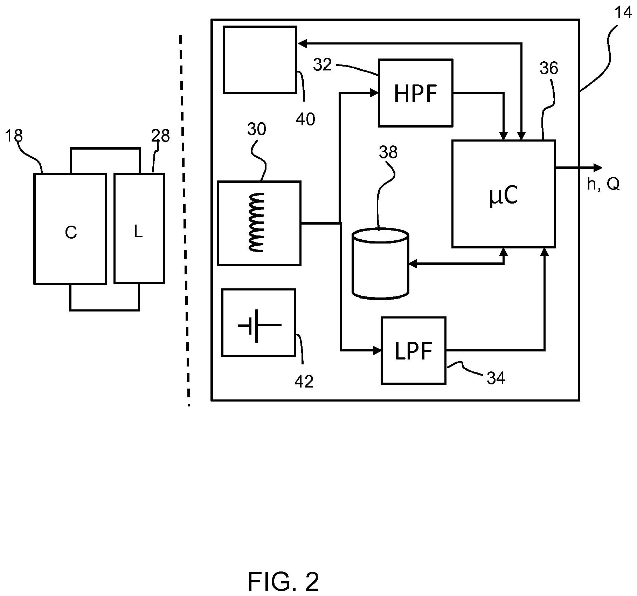

Fig. 2 shows an example of the controller design.

This is the first extreme mentioned above, with a minimum of components implanted into the body. Thus, the controller is outside the body, and it couples wirelessly with the sensor to read the raw sensor signals. The options for this wireless coupling are discussed below.

The sensor forms part of a passive LC electrical circuit, and the sensing translates to an impedance e.g. capacitance variation. The sensor 18 is thus represented by a capacitor in Fig. 2 and there is also an implanted (fixed value) inductor 28 to from the implanted LC circuit. The controller is used to wirelessly readout the capacitance variations.

The external controller 14 then comprises a pick up coil 30 which inductively couples with the passive LC circuit. A method for reading out both slowly and rapidly varying capacitance is for example described in Kim, H.S. et al. "Inductive coupling based wireless sensors for high frequency measurements", Chapter 19 in Smart Sensors for Industrial Applications, ed. Krzysztof Iniewski, CRC Press 2013, pages 305-322, ISBN 978-1-4665-6810-5.

The capacitance variations may be applied to a high pass filter 32 to enable extraction of the high frequency capacitance variations and a low pass filter 34 to enable extraction of the low frequency variations.

A processor 36 then determines the bio-layer thickness h and flow Q. This determination is for example based on a look up table 38.

During use of the sensor, the resonance frequency fᵣₑₛ of the sensor is initially determined from the high frequency capacitance variations.

The free length of the sensor L_{free} = L-h can then be determined from a calibrated relationship fᵣₑₛ = f(L-h). It may be assumed that the resonance frequency only depends on the free length of the sensor.

The bio-layer (plaque) thickness may then be derived as h = L - L_{free}.

The local flow velocity is obtained from the slowly varying capacitance C. It can be assumed that the capacitance C depends on the velocity and the free length of the sensor, C = f(v,:L_{free}).

The look up table 38 thus provides a first mapping between high frequency sensor signals (i.e. the resonance frequency) and changes in length and a second mapping between low frequency sensor signals and a level of flow (with different sub-tables for different values of the length). This look up table may be populated by a calibration routine. Alternatively, algebraic functions may be used to map between sensor readings and the read out parameters.

The local flow velocity can be translated to a volumetric flow rate based on the known cross sectional area of the lumen and an assumed flow type (e.g. laminar, turbulent).

The controller is also provided with a memory 40 for storing either the raw sensor values or the final output information (flow rate and bio-layer thickness) and a local battery 42 is shown (which is relevant if the controller functionality is instead to be part of an implanted device).

If only the bio-film formation capability is needed, only the corresponding measurements and processing may be implemented. This can also be done in case only the flow measurement needs to be implemented. Preferably both of the capabilities are however implemented.

The implementation above has entirely passive components within the implanted part of the system. It is a based on a system in which the exposure to the blood flow induces both high frequency resonant oscillations and low frequency deformation in the sensor in response to the flow.

An alternative approach is to induce vibrations at the resonant frequency via active electrical vibration. An electrical vibration signal, in the form of e.g. a frequency sweep, can be applied to determine the mechanical vibration or resonance frequency. In this case it is required to use an actively driven element such as an EAP (preferably an IPMC). The active driving could be realized based on wireless power transmission or using a local energy source.

By way of example, a first option is to measure (and optionally process) sensor data only on demand for example if the user places a wireless transmitter, which includes wireless powering, above the implant. This may be appropriate if data is only needed for a regular check a few times during the day.

A second option is to continuously collect and record sensing information in the implanted device as well as providing local power for the actuation if actuation is required. In this case, a (rechargeable) battery is used to provide local power. Wireless powering may then be used to charge the battery, for example over night by wearing a charging belt. Furthermore, the wireless coupling may be used to read out the stored data if the implant is not provided with active transmitting functionality. Alternatively, the implant may have locally powered wireless data transmission capability.

Thus it is possible to have an implanted power source or to provide power wirelessly, as well as having different possible levels of data processing and storage in the implanted device.

The frequencies involved will depend on the particular sensor design. However typically the resonance frequency is in the kHz range, for example about 30 kHz. A typical range is 10 kHz to 100 kHz.

The local flow profile can be more accurately determined by actuating the sensor with a DC offset voltage to change the degree of bending of the sensor. The degree of bending determines how far the sensor protrudes into the vessel. For large actuation, the sensor tip will be closer to the wall and the measurement will represent the flow near the wall. The relationship between the capacitance change and the flow for different actuated shapes of the sensor may be calibrated and stored in the lookup table 38 as mentioned above.

Thus, the sensor may be a sensor-actuator which allows simultaneous sensing and actuation. For an IMPC sensor-actuator, this can be achieved by measuring the impedance of the outer electrodes separately to the actuation voltage or adding a high frequency signal to the quasi-dc actuation signal. The approach of combining a DC actuation signal with a high frequency superposed AC signal for sensing is described in detail in WO 2017/036695.

For the most basic passive system of Fig. 2, there is the advantage of the simplicity and hence reliability of the implanted part of the system. The disadvantage is that an external controller is needed to obtain sensor signals. The external controller may be part of an externally worn item so that continuous monitoring is still possible, but this is more intrusive for the user. Alternatively, it may only be desired to perform measurements periodically, e.g. daily, in which case the need to apply the external controller is less of an inconvenience for the user.

As mentioned above, some or all of the controller functionality may instead be provided as part of the implanted device. This provides a more complex implant but it enables continuous monitoring (by which is meant at periodic sampling instants) in a less obtrusive manner. An external controller is then needed only for data download purposes. The continuous monitoring can give detailed information on how bio-film formation occurs over time and at different locations as well as how this influences the blood flow in associated vessels.

The support device diameters 19 typically vary from mm's to cm's depending on the application (peripheral artery disease, coronary artery disease, abdominal aortic aneurysm). The sensor length L may vary accordingly. The sensor length L may be half the stent diameter D in order to capture the bulk of the flow, L = 0.5 D, or more generally between 0.3 D and 0.7 D. The sensor length may instead be even smaller (L << D), for example for measuring a shear rate close to the vessel wall. This is a risk indicator for plaque formation.

In the example, the support device is in the shape of a cylinder with a diameter 19. The device may have an adjustable diameter either to accommodate positioning against the wall of different diameter vessels or to facilitate implantation (see below). Thus the cylinder may have an extended state (used when in operational form) with an extended state diameter comparable to that of the vessel and a collapsed state with a diameter that is smaller than that of the extended state diameter. For example, the diameter may be 10%, 20% 50 % of the extended state diameter. In order to facilitate such collapsing property, the sensor may have a bent shape when the support device is in collapsed state. This bent shape may be actively imposed using an actuation part at the location of mounting of the sensor to the support device. Otherwise, the device can just make use of the ability of the sensor to bend.

Such collapsible devices are known from stents and those skilled in the art will know how to make them. The device of the invention can thus be conveniently added to a stent and check the condition of a stent when it comes to biofilm formation.

The support device can be made of a grid or mesh of material instead of for example a closed surface cylinder. This is known from stent type devices.

The support device does not need to have a cylindrical shape, nor does it need to be for positioning against a wall. After all, placing the device in a flow within a vessel will create conditions of flow near the support device surface that may cause bio-film formation. If not against the wall, there may be more than one sensor device on more than one surface that is in contact with the flow when a device is in use, as will be discussed herein below. Placing it against a wall facilitates fastening and makes sensors at one side against the wall not necessary.

Stent or support device diameters 19 typically vary from mm's to cm's depending on the application (peripheral artery disease, coronary artery disease, abdominal aortic aneurysm). The sensor length L may vary accordingly. The sensor length L may be half the stent diameter D in order to capture the bulk of the flow, L = 0.5 D, or more generally between 0.3 D and 0.7 D. The sensor length may instead be even smaller (L << D), for example for measuring a shear rate close to the vessel wall. This is a risk indicator for plaque formation.

In the example above, the sensor device is in perpendicular position to the length direction 17 and along the diameter 19, thus perpendicular to the cylinder surface. This is a basic implementation. However, the sensor device in use state can have an angle with either or both of the length direction 17 and the diameter direction 19, and thus need not be perpendicular to the cylinder wall. As long as the sensor has a sensing part spaced from the wall.

In the example shown in the Figs. 1A and 1B, there is just one sensor. However, support devices can have more than one sensor at different locations on an area of the support device. This may have for example advantages to obtain information on bio-layer formation distribution around and or along a part of a vessel wall. For example, such formation may not be symmetrical around an artery or vessel wall.

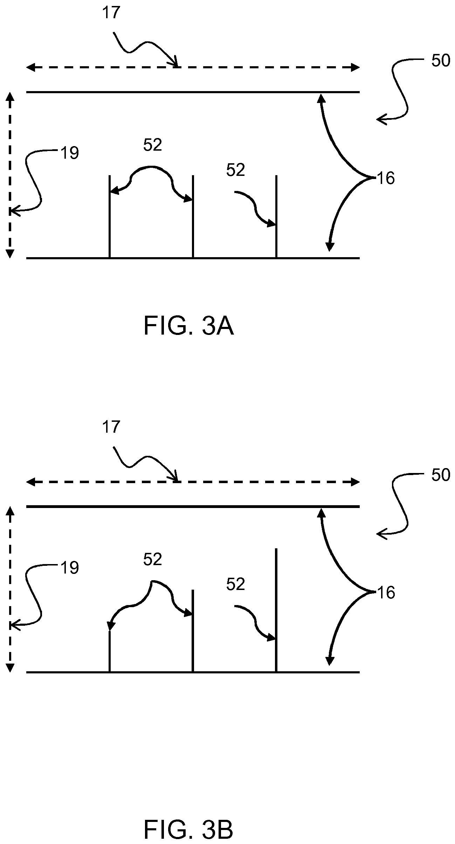

Figs. 3A and 3B show simplified cross-sections of two cylindrical support device 50 each having three sensors 52 along a part of their length 17. In the example of Fig. 3A the sensors 52 are equally long and in Fig. 3B they have different lengths. Each of the lengths may be as defined herein before. The devices have the advantage that the bio-layer and/or flow can be measured along an extension of the device. For example, the formation and/or flow at the left outermost sensor may be different from that at the right outermost one due to for example flow direction in the vessel and thus in the support device. This can be sensed with such configuration. The examples have three sensors each, but any other number of sensors greater than 1 can be used. The sensors of different length may give a different sensor signal at a same bio-layer thickness and flow ranges.

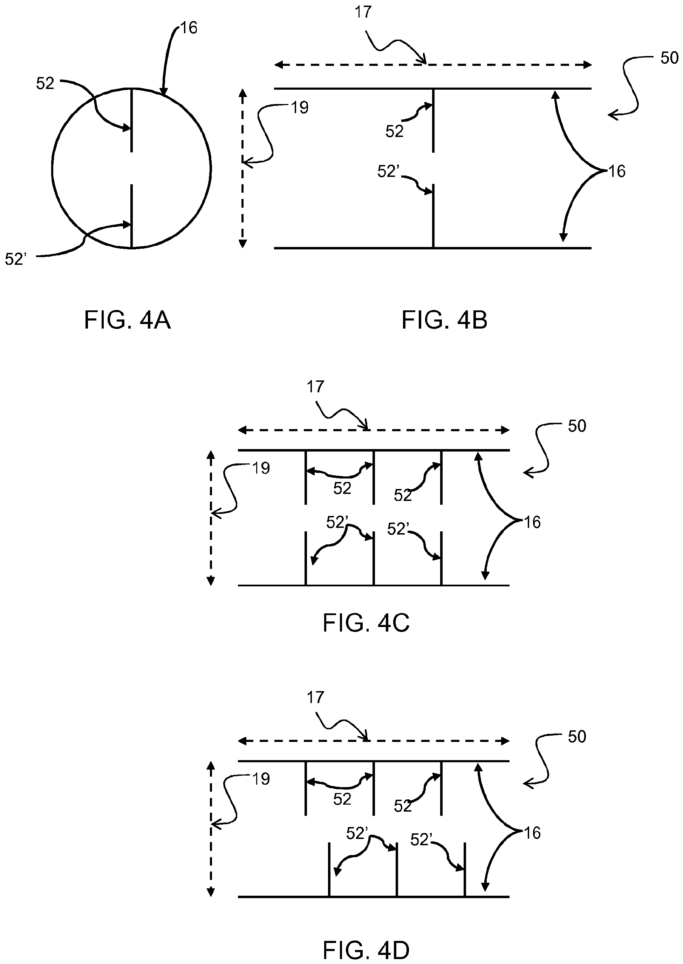

Figs. 4A to 4D show implementations of multiple sensors 52 and 52' at different axial positions (around the circumference) of the support device 50 or perpendicular to the length direction 17. Those of Fig. 4C and 4D also have multiple sensors 52 distributed along the length 17 as described herein before. This configuration with different axial positioned sensors allows monitoring the bio-layer buildup at a length position but at different axial positions of the support. Fig 4D shows multiple of the sensors 52' to be at different length positions as the sensors 52. Hence they sensors at opposite sides may have a length larger than half of the diameter without disturbing each other.

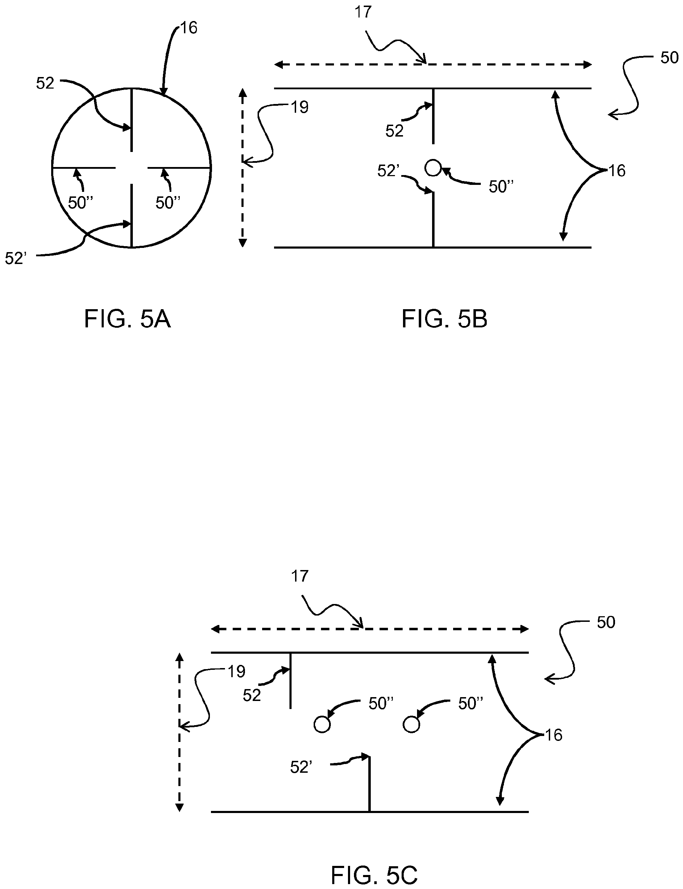

Figs. 5A to 5D show implementations of multiple sensors 52, 52' 50" and 50‴ at axial positions that make an angle of 90 degrees with each other around the circumference of the support device. These may be at the same length position as in Fig. 5B, but then at least two of them are best interrupted (have non-fixed ends) and for all opposing ones, the sum of their lengths is preferably smaller than or equal to the diameter 19. This configuration with different axial positioned sensors allows monitoring the bio-layer buildup at a length position but at different axial positions of the support. Fig. 5C shows the right angle configuration but then distributed along the length direction.

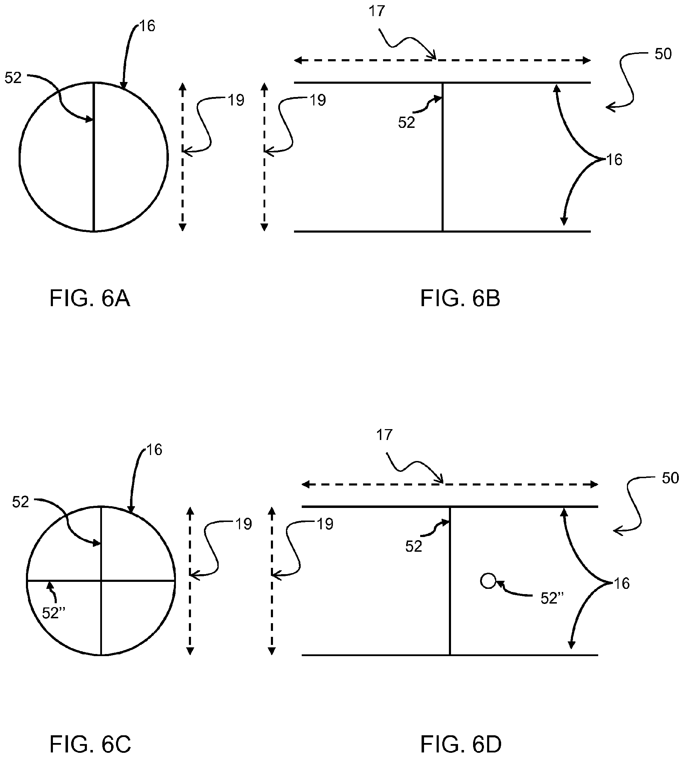

In the above multiple sensor configurations all of the sensors have a free end and a mounted end. However, this need not be the case. Figs. 6A to 6F shows configurations with support devices 16 that have sensors 52 that are mounted with both their long ends to the support device. The double mounting may provide increased robustness and stiffness if needed. After all, a loose sensor in a vessel may not be desirable. Such configuration can also be advantageous in vessels with strong flow or high forces (viscous fluid flow). The principles of sensing and measurement as described for the one end mounted sensors herein above holds true for the double ended sensors too. Flexibility may be different, but this can be accounted for by the controller and design of the sensor can be adjusted to use more flexible materials if needed. The stiffness tailoring may be used define resonance frequencies needed for a particular application.

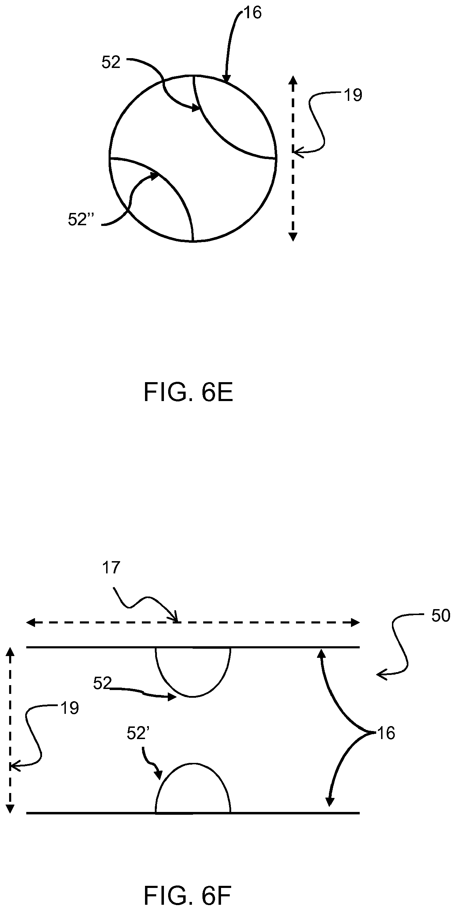

Thus Figs. 6A to 6B show a sensor 52 mounted along the diagonal at both sides of the support device. It can measure the bio-layer growth at opposite ends. Figs. 6C and D shows a similar principle but now for two sensors 52 and 52" at 90 degrees' angle. Fig. 6E shows two sensors 52 and 52" mounted with two ends each at 90 degree mounting locations so that the sensors are in bent configuration in a cross-section of the support device that is perpendicular to the length direction. Fig. 6F shows that such mounting with bending can also be done in the length direction of the support device. The pre-bent sensor configurations can be advantageous in support devices or stents that are in collapsed state before placement in a vessel.

The multiple sensors can be read or addressed by the controller using array techniques or just using multiple controllers. The addressing may be parallel or in series or both.

The support device can be in the form of an implantable that is intended to remain in the body for quite some time such as days, weeks months or even years. However, the device can also be part of for example a catheter which is inserted in the body during treatment and monitoring e.g. in hospital. In all cases materials chosen for the device are in body compatible and parts of the device or system may be coated with specific materials for this. Such materials are known in the field.

If the device is in the form of a support device that is implantable such as a stent, then before and during implantation, the stent with its integral sensor is preferably folded in a delivery catheter. When the stent is released the sensor unfolds itself in its upright position.

Several unfolding methods are possible, some of which make use of the ability of electroactive polymer devices to function both as actuators and sensors.

In a first approach, the sensor unfolds itself due to its elasticity, with the sensor folded flat against the stent in a delivery catheter against this elastic bias.

In a second approach, the sensor has a shape memory material backing foil which changes its shape from flat to upright position when activated at body temperature.

In a third approach, the sensor is connected to a slightly pre-curved stent strut, which twists out of plane (inward of the blood vessel) when the stent expands.

In a fourth approach, the sensor itself is a slightly pre-curved beam, which twists out of plane (inward of the blood vessel) when the stent expands.

In a fifth approach, the sensor is unfolded in a non-actuated resting state. It may then be actuated during placement to lay flat. After placement the actuation voltage may be released and the sensor can operate as a passive sensor.

In a sixth approach, the sensor is held flat with a latch. When the stent has been placed, the electroactive polymer sensor-actuator is actuated to deform and the sensor-actuator is released from the latch. Alternatively, the latch itself may be an actuator device.

As described, the examples above show single sensor ones, but also multiple sensor systems. When multiple sensors are present differences in response may be analyzed, giving additional info on area specific (different layer formation at different locations around the system) bio-layer formation. For example, endothelium overgrowth would be more homogenous than the formation of plaque. From monitoring the differences, the build-up of plaque vs. endothelium overgrowth can be assessed. There may be multiple sensors around the circumference and/or in the axial direction and configurations as described herein before can be used.

The multiple sensors can also be implemented for measuring in different flow ranges and to this end they may for example be implemented with different stiffness.

Contamination of the sensor may influence the resonant frequency, although since the sensor moves in the blood flow the risk of contamination is limited. However, in order to further reduce the risk of contamination, the sensor may be vibrated intermittently with ultrasonic frequencies (e.g. in the range 10-100 kHz) to actively clean the sensor.

As mentioned above, there are various options for wireless connection to the implanted sensor, either to provide a remote source of power, or to provide a communication channel or both.

In general, an implant, whether passive or active, may be powered in many ways. Depending on the functionality and operation mode of the implant, different requirements for the energy source are present.

For a continuous active function, such as a requirement for active mechanical actuation in order to generate an output signal, there is a higher energy requirement than for a passive temporally limited (e.g. on-demand) function, such as the occasional read out of an active sensor. However, in both cases, there is a need for either a wired connection to a local power source, or a wireless coupling to a power transmitter.

Delivering electrical power to medical implants for powering or communication is a topic which is well-described in literature.

Comprehensive reviews of power aspects for implantable medical devices are given in B. A. Achraf, A. B. Kouki and C. Hung, "Power Approaches for Implantable Medical Devices," sensors, no. 28889-28914; doi:10.3390/s151128889, 2015, J. Lee, J. Jang and Y.-K. Song, "A review on wireless powering schemes for implantable microsystems in neural engineering applications," Biomed Eng Letters, no. DOI 10.1007/s13534-016-0242-2, pp. 6:205-215, 2016, A. Kim, M. Ochoa, R. Rahim and B. Ziaie, "New and Emerging Energy Sources for Implantable Wireless Microdevices," IEEE: SPECIAL SECTION ON NANOBIOSENSORS, no. 10.1109/ACCESS.2015.2406292, 2014, and K. N. Bocan and E. Sejdi'c, "Adaptive Transcutaneous Power Transfer to Implantable Devices: A State of the Art Review," sensors, vol. 16, no. doi:10.3390/s16030393, p. 393, 2016.

Any of these solutions may be used to power the implant, and some approaches will be discussed below.

A first approach is to provide a wired power source as part of the implant. In this case, some or all of the functionality shown in Fig. 2 may be incorporated into the implanted part of the system. A wired power source may be an ordinary battery (non-rechargeable or rechargeable) such as shown as 42, directly connected to the implant or to its operating electronics. However, since implants usually will be worn over a long period of time, a high capacity and high energy density battery would be of benefit. The power density of (re-chargeable) batteries is expected to grow further making them increasingly suitable for long term monitoring functions.

Instead of conventional batteries, bio-fuel cells or nuclear batteries may be applicable. Another alternative power source, which is very similar to a battery, is a super capacitor, which is a capacitor having an extremely high capacitance and a very low self-discharge characteristic.

Energy harvesters may instead be used to operate any implant. Accordingly, a power generator could for example be operated by human body energy such as motion of an extremity but also motion of an inner organ or any dynamics resulting from a fluid flow (blood in an artery) or gas (air in a lung). The power generator may be able to store energy in a super capacitor or re-chargeable battery, and/or be able to directly operate an implant.

An energy harvester does not necessarily need to be in close vicinity to the implant itself but could also be spatially separated. A wired connection may be used between them. Also in the field of energy harvesters, efforts are being made to make them smaller and more efficient in order to make them more attractive as an internal (and everlasting) energy source for medical devices.

Wireless energy transmission systems may be classified according to the physical coupling mechanism, which can be either capacitive, inductive (magnetic) or electromagnetic. All three mechanisms have their own pros and cons and preferred applications. In general, the performance of each approach depends very much on specific boundary conditions such as e.g. the size of the transmitter- and receiver-element (which can be a plate, an inductor or an antenna) and the distance and medium between both elements, as well as their orientation with respect to each other.

An additional smart feature of all wireless power systems is the intrinsic ability of a bidirectional data communication between a transmitter and a receiver.

In applications where low energy levels at short distances need to be transmitted, capacitive coupling may be used. Low to medium power levels at medium to long range may be preferably realized via an electromagnetic coupling. Highest power levels at short distances may be transmitted via an inductive coupling, making use of magnetic fields.

As mentioned above, the most basic approach only enables sensor data to be gathered when the external controller is present. However, using such a wireless powering technique would not necessarily imply the need to wear such a transmitter continuously to perform the intended use of the implant. For example, an implant may only need to be operated during certain treatments (in e.g. a hospital) or it may only need to be activated at predefined moments in time (e.g. morning, afternoon, evening).

An alternative use case would be to use such a wireless transmitter overnight, to charge an implanted power source, which would be used to operate an implant during the day. This is a hybrid approach where there is a local energy supply so sensor data can be gathered and stored in memory without an external controller in place, but it has a short duration so needs recharging periodically.

The implanted wireless receiver unit and the implanted sensor may be spatially separated from each other. For example, the receiving element, e.g. a receiver inductance may be located directly underneath the skin, in order to realize a strong coupling between the transmitter and receiver and thus to maximize the energy transmission efficiency and to minimize the charging time of an implanted battery. Of course, this would require a more involved implantation procedure than if the implanted elements are fully integrated into the stent (or other support structure).

There are also options which do not rely on electrical energy to realize a wireless energy transmission system, in particular making use of optical, ultrasonic or mechanical pressure waves.

The example above makes use of a single beam for the sensor. There are alternative designs, such as multiple radial webs. They may even surround the stent to form a mesh.

As discussed above, a controller performs the data processing. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

As mentioned above, the sensor may be implemented using an electroactive polymer (EAP) device. EAPs are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications. An EAP device can be used in any application in which a small amount of movement of a component or feature is desired, based on electric actuation or for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nafion^{®} and Flemion^{®}.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

For the sensing functionality, the use of capacitance change is discussed above, in particular in connection with an ionic polymer device. For field driven systems, an impedance and/or capacitance change can also be measured directly or by measuring changes in electrode resistance as a function of strain in the sensor.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. O₂, NO₂), making CNTs usable as gas detectors.

Sensing may also be based on force measurements and strain detection. Dielectric elastomers, for example, can be easily stretched by an external force. By putting a low voltage on the sensor, the strain can be measured as a function of voltage (the voltage is a function of the area).

The main example of interest is for monitoring a subject after insertion of a stent. Stent placement may be in a coronary artery, with the risk that the stent becomes blocked again due to restenosis or due to the formation of scar tissue. Stent placement may instead be in the lower leg to open-up a blocked artery caused by peripheral artery disease. The blood flow pattern is then disrupted and there is a risk that in the treated artery the blood flow suddenly increases due to which another artery suddenly receives less flow ("vascular steal"). Flow monitoring is able to determine this.

The invention may be applied to stents as mentioned above, but also to stent grafts, heart valves, coronary arterial bypass grafts and shunts. Some of these are discussed below. Furthermore, the sensor design described above may be used only for intravascular flow measurement or only for bio-layer monitoring. There are various applications where flow measurement is of interest and/or bio-layer formation is possible.

During Coronary Artery Bypass Graft (CABG) surgery, there is a need to know if the blood flow in the bypass is sufficient. This can be monitored for example with a cuff-like device which is placed around the graft during surgery. In general, the prospect for recovery is good: about 90% of patients experience significant improvements after coronary artery bypass graft surgery and for most people, the graft remains open for about 10-15 years. However, coronary artery bypass surgery does not prevent coronary artery disease from recurring. In 5-10% of coronary artery bypass graft surgeries, the bypass graft stops supplying blood to the bypassed artery within one year. About 40% of patients have a new blockage within 10 years after surgery and require a second bypass, change in medication, or an interventional procedure.

In order to provide reassurance, early warning, or optical (minimum) medication it desired to monitor the arterial bypass graft after the surgery, to check whether the blood flow does not drop below a certain value and to timely intervene if necessary.

Monitoring may also be used for active surveillance of indolent cancer. Cancer treatment can have negative side effects, for instance with prostate cancer. In such cases it might be considered to monitor the tumor at regular intervals with doctor visits and to treat only when the tumor starts to grow. Slow prostate growth may allow such a strategy. However, if there is too much time between the doctor visits this may give unrecorded metastasis. In this case continuous monitoring of the blood flow in and out the prostate can provide an indication for the tumor growth.

The "May Thurner syndrome" (MTS) is a condition in which compression of the common venous outflow tract of the left lower extremity may cause discomfort, swelling, pain or blood clots, called deep venous thrombosis (DVT). The problem is that the blood is not transported back to the upper body. Often it is unclear if or when stenting is needed. A temporary or a small unobtrusive sensor could provide decision support. This is important because MTS often concerns young people and a stent stays for life; the stent may also cause damage or deformation of this important vessel. A sensor may also be used to enable diagnosis during walking (whereas diagnosis in the hospital is in lying position). A small and local sensing implant might be acceptable if it can prevent unnecessary implantation of a big stent.

It may also be of interest to measure the flow in a dialysis shunt. A shunt is an artificial loop (plastic tube) between an artery and a vein in the forearm with an access point to an external dialysis circuit. For timely intervention it is desirable to know or predict when the flow drops below a certain level. Currently the flow is monitored externally for example three times each week during dialysis based on the pre-pump arterial pressure. Between dialysis, the shunt resistance can be subjectively monitored by the patient via tactile methods (sense the vibration of blood going through the arm), or by a physician using a stethoscope. However, clotting or shunt compression for example during sleep might be acute and continuous quantitative flow monitoring would safeguard the shunt.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A system, comprising:
an intra-vascular support device (16) having a length direction, for positioning against a vessel wall (10) in use, with its length direction aligned with a vessel direction;
a sensor device (18, 52) mounted to the intra-vascular support device (16), and projecting from the intra-vascular support device (16) into a vessel content, the sensor device (18, 52) consisting of a deformable part that is mounted to the intra-vascular support device (16) such that when a flowing medium flows along the support device (16), the deformable part at least partly extends perpendicularly to the length direction of the intra-vascular support device (16) into the flowing medium, the sensor device (18, 52) being adapted to generate a sensor signal which is dependent on a deformation or deformability of the deformable part; and
a controller (14) adapted to:
receive the sensor signal; and
determine from the received sensor signal an indication of a thickness of a bio-layer formation on the intravascular support device (16);
determine a length of the deformable part of the sensor device (18, 52) at a desired time based on determining a resonant frequency of the sensor device (18, 52);
wherein the indication of a thickness of the bio-layer is derived from the determined length.

2. A system as claimed in claim 1, wherein the intra-vascular support device (16) and the sensor device (18, 52) are part of a catheter or an implantable unit.

3. A system as claimed in claim 1 or 2, wherein the controller (14) is adapted to determine from the received sensor signal, an indication for a level of flow of the flowing medium.

4. A system as claimed in any of the previous claims, wherein the intra-vascular support device (16) is for positioning against a vessel wall and/or comprises a tubular shape when in use, the tubular shape defining a hollow internal space and the deformable part of the sensor device being located within the internal hollow space.

5. A system as claimed in any of the previous claims, wherein the sensor device (18, 52) includes a first end and a second end, with the deformable part being located between the first end and the second end, the first end being mounted to the intra-vascular support device (16) at a first position, and the second end being mounted to the intra-vascular support device (16) at a second position different from the first position.

6. A system as claimed in claim 4, wherein the intra-vascular support device (16) comprises a tubular shape, and the first and second positions are diametrically opposite to each other when the support device is in use.

7. A system as claimed in any one of claims 1 to 4, wherein the sensor device (18, 52) includes a first end and a second end, with the deformable part located between the first and second end, the first end being a freely movable and the second end being mounted to the intra-vascular support device (16).

8. A system as claimed in claim 7, wherein the intra-vascular support device (16) comprises a tubular shape when in use with a first diameter, and the sensor device (18, 52) has a length measured from the first end to the second end which is smaller than or equal to the first diameter, and preferably is between 0.4 and 0.7 times the first diameter.

9. A system as claimed in any preceding claim, wherein the sensor device (18, 52) comprises an electrical impedance, and in particular an electrical capacitance, which is dependent on a deformation of the deformable part of the sensor device.

10. A system as claimed in any preceding claim, wherein the controller (14) is adapted to determine the resonant frequency based on electrical capacitance changes of the sensor device (18, 52) in a frequency range above a frequency range of the capacitance variation caused by a level of flow (20) being monitored.

11. A system as claimed in claim 10, wherein the controller (14) is adapted to determine the resonant frequency based on resonance induced by the flow (20) being monitored.

12. A system as claimed in claim 10, wherein the controller (14) is further adapted to actuate the sensor device (18, 52), and to determine the resonant frequency based on resonance induced by the actuation.

13. A system as claimed in any preceding claim, wherein the controller (14) is further adapted to actuate the sensor device (18, 52) at an ultrasound frequency.

14. A system as claimed in any preceding claim, wherein the controller (14) is adapted to determine a level of flow based on the amplitude of the sensor signal.

15. A system as claimed in any preceding claim, further comprising a look-up table (38) for providing a first mapping between sensor signals and changes in length, and a second mapping between sensor signals and a level of flow.

16. A system as claimed in any preceding claim, wherein the sensor device (18, 52) has a non-deployed folded state for use during implantation of the system, and a deployed unfolded state for subsequent use in monitoring a subject.

## Patentansprüche

1. Ein System, das Folgendes umfasst:
ein längs ausgerichtetes intravaskuläres Trägergerät (16) das so an einer Gefäßwand (10) positioniert wird, dass die Längsrichtung an der Gefäßrichtung ausgerichtet ist;
ein am intravaskulären Trägergerät (16) angebrachte Sensorgerät (18, 52), das vom intravaskulären Trägergerät (16) in den Gefäßinhalt hineinragt, wobei das Sensorgerät (18, 52) aus einem verformbaren Teil besteht, der so am intravaskulären Trägergerät (16) angebracht ist, dass sich der verformbare Teil zumindest teilweise senkrecht zur Längsrichtung des intravaskulären Trägergeräts (16) in ein fließendes Medium erstreckt, wenn entlang des Trägergeräts (16) ein fließendes Medium fließt, wobei das Sensorgerät (18, 52) ein Sensorsignal erzeugt, das von einer Verformung oder der Verformbarkeit des verformbaren Teils abhängig ist; und
eine Steuerung (14), die folgende Schritte durchführt:
Abrufen des Sensorsignals; und
Ermitteln einer Angabe zur Dicke der Bioschicht-Bildung auf dem intravaskulären Trägergerät (16) anhand des empfangenen Sensorsignals;
Ermitteln der Länge des verformbaren Teils des Trägergeräts (18, 52) zu einem gewünschten Zeitpunkt anhand des Ermittelns der Resonanzfrequenz des Sensorgeräts (18, 52);
wobei die Angabe zur Dicke der Bioschicht aus der ermittelten Länge abgeleitet wird.

2. Ein System gemäß Anspruch 1, wobei das intravaskuläre Trägergerät (16) und das Sensorgerät (18, 52) Teil eines Katheters oder einer implantierbaren Einheit sind.

3. Ein System gemäß Anspruch 1 oder 2, wobei die Steuerung (14) anhand des abgerufenen Sensorsignals eine Angabe zum Strömungspegel des fließenden Mediums ermittelt.

4. Ein System gemäß einem der vorherigen Ansprüche, wobei das intravaskuläre Trägergerät (16) an einer Gefäßwand positioniert wird und/oder im Gebrauch eine röhrenförmige Form aufweist, wobei die röhrenförmige Form einen hohlen Innenraum definiert und der verformbare Teil des Sensorgeräts innerhalb des inneren Hohlraums angeordnet ist.

5. Ein System gemäß einem der vorherigen Ansprüche, wobei das Sensorgerät (18, 52) ein erstes und ein zweites Ende aufweist, wobei der verformbare Teil zwischen dem ersten Ende und dem zweiten Ende angeordnet ist, wobei das erste Ende an einer ersten Position am intravaskulären Trägergerät (16) und das zweite Ende an einer zweiten, von der ersten Position abweichenden Position am intravaskulären Trägergerät (16) angebracht ist.

6. Ein System gemäß Anspruch 4, wobei das intravaskuläre Trägergerät (16) eine röhrenförmige Form aufweist und sich die erste und die zweite Position einander diametral gegenüberliegen, wenn das Trägergerät verwendet wird.

7. Ein System gemäß einem der Ansprüche 1 bis 4, wobei das Sensorgerät (18, 52) ein erstes Ende und ein zweites Ende aufweist, und wobei der verformbare Teil zwischen dem ersten und dem zweiten Ende angeordnet ist, wobei das erste Ende frei beweglich ist und das zweite Ende am intravaskulären Trägergerät (16) befestigt wird.

8. Ein System gemäß Anspruch 7, wobei das intravaskuläre Trägergerät (16) im Gebrauch eine röhrenförmige Form mit einem ersten Durchmesser aufweist, und wobei das Sensorgerät (18, 52) eine vom ersten bis zum zweiten Ende gemessene Länge aufweist, die höchstens dem ersten Durchmesser entspricht und vorzugsweise das 0,4- bis 0,7-Fache des ersten Durchmessers beträgt.

9. Ein System gemäß einem der vorhergehenden Ansprüche, wobei das Sensorgerät (18, 52) eine elektrische Impedanz und hierbei insbesondere eine elektrische Kapazität aufweist, die von der Verformung des verformbaren Teils des Sensorgeräts abhängig ist.

10. Ein System gemäß einem der vorhergehenden Ansprüche, wobei die Steuerung (14) die Resonanzfrequenz anhand der Veränderungen der elektrischen Kapazität des Sensorgeräts (18, 52) in einem Frequenzbereich ermittelt, der oberhalb des Frequenzbereichs der durch den überwachten Strömungspegel (20) verursachten Kapazitätsabweichung liegt.

11. Ein System gemäß Anspruch 10, wobei die Steuerung (14) die Resonanzfrequenz anhand der durch die überwachte Strömung (20) induzierten Resonanz ermittelt.

12. Ein System gemäß Anspruch 10, wobei die Steuerung (14) zudem das Sensorgerät (18, 52) betätigt und die Resonanzfrequenz anhand der durch die Betätigung induzierten Resonanz ermittelt.

13. Ein System gemäß einem der vorhergehenden Ansprüche, wobei die Steuerung (14) das Sensorgerät (18, 52) zudem mit einer Ultraschallfrequenz betätigt.

14. Ein System gemäß einem der vorhergehenden Ansprüche, wobei die Steuerung (14) den Strömungspegel anhand der Amplitude des Sensorsignals ermittelt.

15. Ein System gemäß einem der vorhergehenden Ansprüche, das zudem eine Suchtabelle (38) zum Bereitstellen einer ersten Zuordnung von Sensorsignalen zu Längenänderungen sowie einer zweiten Zuordnung von Sensorsignalen zu einem Strömungspegel umfasst.

16. Ein System gemäß einem der vorhergehenden Ansprüche, wobei das Sensorgerät (18, 52) während des Implantierens des Systems einen nicht operativen gefalteten Zustand sowie bei der anschließenden Verwendung zur Überwachung des Patienten einen operativen entfalteten Zustand aufweist.

## Revendications

1. Système comprenant :
un dispositif de support intravasculaire (16) ayant une direction de longueur, conçu pour être positionné contre une paroi de vaisseau (10) lors de l'utilisation, avec la direction de longueur alignée avec une direction du vaisseau ;
un dispositif capteur (18, 52) installé sur le dispositif de support intravasculaire (16), et faisant saillie depuis le dispositif de support intravasculaire (16) dans un vaisseau, le dispositif capteur (18, 52) consistant en une partie déformable qui est installée sur le dispositif de support intravasculaire (16) de sorte que lorsqu'un fluide en écoulement s'écoule le long du dispositif de support (16),
la partie déformable au moins partiellement s'étend perpendiculairement à la direction de la longueur du dispositif de support intravasculaire (16) dans le fluide en écoulement, le dispositif capteur (18, 52) étant conçu pour générer un signal capteur qui est dépendant d'une déformation ou d'une déformabilité de la partie déformable ; et
un contrôleur (14) conçu pour :
recevoir le signal de capteur : et
déterminer à partir du signal de capteur reçu une indication d'une épaisseur d'une formation de bio-couche sur le dispositif de support intravasculaire (16) ;
déterminer une longueur de la partie déformable du dispositif capteur (18, 52) à un temps souhaité sur détermination d'une fréquence de résonance du dispositif capteur (18, 52) ;
dans lequel l'indication d'une épaisseur de la bio-couche est dérivée de la longueur déterminée.

2. Système selon la revendication 1, dans lequel le dispositif de support intravasculaire (16) et le dispositif capteur (18, 52) font partie d'un cathéter ou d'une unité implantable.

3. Système selon la revendication 1 ou 2, dans lequel le contrôleur (14) est conçu pour déterminer à partir du signal de capteur reçu, une indication d'un niveau de flux du fluide en écoulement.

4. Système selon l'une quelconque des revendications précédentes, dans lequel le dispositif de support intravasculaire (16) est conçu pour être positionné contre une paroi de vaisseau et/ou comprend une forme tubulaire lors de l'utilisation, la forme tubulaire définissant un espace interne creux et la partie déformable du dispositif capteur étant placée à l'intérieur de l'espace interne creux.

5. Système selon l'une quelconque des revendications précédentes, dans lequel le dispositif capteur (18, 52) comprend une première extrémité et une seconde extrémité, avec la partie déformable étant placée entre la première extrémité et la seconde extrémité, la première extrémité étant installée sur le dispositif de support intravasculaire (16) à une première position, et la seconde extrémité étant installée sur le dispositif de support intravasculaire (16) à une seconde position différente de la première position.

6. Système selon la revendication 4, dans lequel le dispositif de support intravasculaire (16) comprend une forme tubulaire, et la première et la seconde position sont diamétralement opposées l'une à l'autre lorsque le dispositif de support est utilisé.

7. Système selon l'une quelconque des revendications 1 à 4, dans lequel le dispositif capteur (18, 52) comprend une première extrémité et une seconde extrémité, avec la partie déformable placée entre la première et la seconde extrémité, la première extrémité étant une extrémité mobile et la seconde extrémité étant installée sur le dispositif de support intravasculaire (16).

8. Système selon la revendication 7, dans lequel le dispositif de support intravasculaire (16) comprend une forme tubulaire lors de l'utilisation avec un premier diamètre, et le dispositif capteur (18, 52) a une longueur mesurée de la première extrémité à la seconde extrémité qui est plus petite que le premier diamètre ou égale à celui-ci, et de préférence est entre 0,4 et 0,7 fois le premier diamètre.

9. Système selon l'une quelconque des revendications précédentes, dans lequel le dispositif capteur (18, 52) comprend une impédance électrique, et en particulier une capacitance électrique, qui est dépendante d'une déformation de la partie déformable du dispositif capteur.

10. Système selon l'une quelconque des revendications précédentes, dans lequel le contrôleur (14) est conçu pour déterminer la fréquence de résonance sur la base des modifications de capacitance électrique du dispositif capteur (18, 52) dans une plage de fréquence au-dessus d'une plage de fréquence de la variation de capacitance causée par un niveau de flux (20) sous surveillance.

11. Système selon la revendication 10, dans lequel le contrôleur (14) est conçu pour déterminer la fréquence de résonance sur la base de la résonance induite par le flux (20) sous surveillance.

12. Système selon la revendication 10, dans lequel le contrôleur (14) est en outre conçu pour actionner le dispositif capteur (18, 52) et pour déterminer la fréquence de résonance sur la base de la résonance induite par l'actionnement.

13. Système selon l'une quelconque des revendications précédentes, dans lequel le contrôleur (14) est en outre conçu pour actionner le dispositif capteur (18, 52) à une fréquence à ultrasons.

14. Système selon l'une quelconque des revendications précédentes, dans lequel le contrôleur (14) est conçu pour déterminer un niveau de flux sur la base de l'amplitude du signal de capteur.

15. Système selon l'une quelconque des revendications précédentes, comprenant en outre une table de référence (38) permettant de fournir un premier mappage entre les signaux de capteur et les changements de longueur, et un second mappage entre les signaux de capteur et un niveau de flux.

16. Système selon l'une quelconque des revendications précédentes, dans lequel le dispositif capteur (18, 52) a un état plié non déployé pour être utilisé durant l'implantation du système, et un état déplié déployé pour être utilisé ultérieurement dans la surveillance d'un sujet.
